# EUROPEAN PATENT SPECIFICATION

(11) **EP 4 587 106 B1**
(45) Date of publication and mention of the grant of the patent: **01.07.2026**
(21) Application number: 23761938.2
(22) Date of filing: 29.08.2023
(51) Int. Cl.: A61N 1/05

(54) **ACTIVE FIXATION CARDIAC ELECTRODE LEAD**
HERZELEKTRODENLEITUNG MIT AKTIVER FIXIERUNG
FIL CONDUCTEUR D'ÉLECTRODE CARDIAQUE À FIXATION ACTIVE

(30) Priority: 15.09.2022 EP 22195784
(43) Date of publication of application: 23.07.2025
(73) Proprietor: BIOTRONIK SE & Co. KG, 12359 Berlin (DE)
(72) Inventor: YEO, Wee Ping, Singapore 570408 (SG); HINRICHSEN, Lisa, Singapore 267952 (SG); KOLBERG, Gernot, 12161 Berlin (DE)
(74) Representative: Biotronik Corporate Services SE
(86) International application number: PCT/EP2023/073600
(87) International publication number: WO 2024/056360

(56) References cited:
- WO-A1-2008/140376
- US-A1- 2003 065 374
- US-A1- 2012 078 336
- US-B1- 6 285 903

## Description

The invention relates to an active fixation cardiac electrode lead. In particular, the invention relates to an active fixation cardiac electrode lead for use with a cardiac pacemaker or an implantable cardioverter/defibrillator or a combination thereof.

Electrode leads are e.g. used in combination with implantable medical devices like cardiac pacemakers or implantable cardioverter/defibrillator and serve for conducting electrical energy to an intracardiac location of a heart to be stimulated or defibrillated. The electrode may be specifically designed to be placed in a chamber of a heart like the right atrium or the right ventricle.

Typically, such electrode lead comprises one or more electrodes close to a distal end of the electrode lead. Each electrode is electrically connected to a connector at the proximal end of the electrode lead via an electrical conductor. The connector serves for connecting the electrode lead to the implantable medical device. In order to keep the distal end of the electrode lead in a fixed position within the heart, active fixation means may be provided at the electrode lead's distal end. A typical active fixation means is a screw-in fixation helix similar to a miniature corkscrew.

In a typical embodiment, the fixation helix is movably placed in a housing at the electrode lead's distal end. The fixation helix can be extended out of the housing's distal end and retracted into an inner space enclosed by the housing by rotating the fixation helix around an electrode lead's longitudinal axis.

During anchoring of the electrode lead in its destination tissue, it is useful for the medical practitioner to know if the fixation helix is turning and to know a total number of revolutions the fixation helix has turned in order to achieve secure anchoring of the fixation helix in the destination tissue. Due to the fact that the electrode is not completely rotationally rigid, the medical practitioner does not know how many rotations the distal end of the electrode lead has been turned. It is therefore helpful for the medical practitioner to observe on an X-ray image whether or not the distal end of the electrode lead is turning.

While it is possible for the medical practitioner to observe the fixation helix to assess the rotation of the tip due to the fixation helix structure shifting axially, it can hardly be seen because the fixation helix is very small and moves considerably in the beating heart.

WO2008/140376 discloses an active fixation cardiac electrode lead.

It is therefore an object of the present invention to provide an improved active fixation cardiac electrode lead that enables the medical practitioner to observe a turning of the fixation helix and consequently to count a number of helix revolutions carried out.

The object is solved by an active fixation cardiac electrode lead having the features of claim 1. Further developments and advantageous embodiments are defined in the dependent claims.

The present invention provides an active fixation cardiac electrode lead. The electrode lead comprises a fixation helix movably or non-movably placed in a housing of the electrode lead at a distal end of said electrode lead.

Furthermore, the electrode lead comprises a first portion of the electrode lead having a first asymmetrical shape and a first radiopacity, and a second portion of the electrode lead having a second asymmetrical shape and a second radiopacity. The second radiopacity differs from said first radiopacity, wherein the first portion of the electrode lead and/or the second portion of the electrode lead are adapted to indicate a rotational position of the electrode lead on an x-ray image during implantation of the electrode lead.

It is an idea of the present invention to provide an electrode lead being built from at least two asymmetrically shaped materials having different radiopacities. In doing so, the shape of each material portion functions as an x-ray marker enabling a medical practitioner to observe a rotational position of the electrode lead on an x-ray image.

X-ray markers are well known in the pacemaker industry. They are used to indicate distances in the human body. It is therefore advantageous to provide a marker indicating the rotation of a lead during implantation. Especially when screwing a lead into the septum when the helix is required to go deeper than a typical two turns to reach the conducting system of the heart, the electrode lead according to the present invention advantageously provides a visual control.

According to an aspect of the invention, the first asymmetrically shaped portion of the electrode lead or the second asymmetrically shaped portion of the electrode lead is integrally formed with the fixation helix or the housing. The part can thus advantageously be die cast and provides a high structural stability.

According to a further aspect of the invention, the first asymmetrical shape of the first portion and the second asymmetrical shape of the second portion are joined together to form the symmetrically, in particular a cylindrically shaped electrode lead. The first portion and the second portion are thus counterparts that are shaped to fit together in a complementary manner.

According to a further aspect of the invention, the first portion of the electrode lead is formed of at least one of platinum, a platinum-iridium alloy, silver, gold, iron, barium, strontium, cobalt, nickel, chromium, and tungsten. Said materials advantageously provide a high level of radiopacity, thus enabling an x-ray contrast on the x-ray image.

According to a further aspect of the invention, the fixation helix is rotatable or fixed relative to the housing of the electrode lead. A suitable helix for a particular application can thus be advantageously provided.

According to a further aspect of the invention, the first portion of the electrode lead and/or the second portion of the electrode lead surround an electrode body. The electrode body is thus securely enclosed by the electrode lead and can thus not be stripped away during and/or after insertion to the destination tissue.

According to a further aspect of the invention, the first portion of the electrode lead and/or the second portion of the electrode lead has an asymmetrical length. This facilitates observation of the rotational position of the electrode lead during insertion to the destination tissue.

According to a further aspect of the invention, the electrode lead comprises a sensor configured to detect a number of revolutions of the fixation helix. The sensor can thus provide a signal to alert the medical practitioner when a predetermined number of rotations have been detected.

According to a further aspect of the invention, a first longitudinal section of the first portion of the electrode lead and the second portion of the electrode lead has a symmetrical, in particular cylindrical shape, and wherein a second longitudinal section of the first portion of the electrode lead and the second portion electrode lead has an asymmetrical shape. The first portion thus surrounds the lead body and the second portion advantageously acts as an x-ray marker to accurately detect a rotational position of the electrode lead.

According to a further aspect of the invention, the first portion of the electrode lead and/or the second portion of the electrode lead comprises a first ring shaped section arranged at a distal end of the electrode lead and a second section having a symmetric, in particular semi-cylindrical cutout. The first ring shaped section thus surrounds the lead body and the second section advantageously acts as an x-ray marker to accurately detect a rotational position of the electrode lead.

According to a further aspect of the invention, the first portion of the electrode lead and/or the second portion of the electrode lead comprises a first ring shaped section arranged at a longitudinal mid-section of the electrode lead and a second section having an asymmetric cutout arranged from the first ring shaped section to each distal end of the first portion of the electrode lead and/or the second portion of the electrode lead respectively. The medical practitioner can thus observe how the second section moves around the electrode body when the electrode rotates.

According to a further aspect of the invention, the first portion of the electrode lead and/or the second portion of the electrode lead comprises a first ring shaped section arranged at a distal end of the electrode lead and a second section comprising a rod extending in a longitudinal direction of the electrode lead. This variant provides more information to the medical practitioner to better distinguish between orientation angles of 90° and 270° of the electrode lead.

According to a further aspect of the invention, the first portion of the electrode lead and/or the second portion of the electrode lead comprises a first ring shaped section arranged at a distal end of the electrode lead and a second substantially elliptical ring-shaped section being oriented oblique to a longitudinal axis of the electrode lead. Since the inclined projection provides more information, angles of 270°, 0°, 90° and 180° can be distinguished easily.

According to a further aspect of the invention, the first portion of the electrode lead and/or the second portion of the electrode lead comprises a substantially elliptical ring-shaped section, a longer axis of which is oriented oblique to the longitudinal axis of the electrode lead. Since the elliptic ring also surrounds the lead body, the small ring is not necessary. The projection of this x-ray contrast component is furthermore easy to interpret for the medical practitioner.

According to a further aspect of the invention, the first portion of the electrode lead and/or the second portion of the electrode lead comprises a first section being formed by the fixation helix and a second section being integrally formed with the fixation helix, said second section having an asymmetric cutout arranged from the first section to a distal end of the first portion of the electrode lead and/or the second portion of the electrode lead. The x-ray component which surrounds the lead body is combined with a helix for lead fixation.

For a more complete understanding of the present invention and advantages thereof, reference is now made to the following description taken in conjunction with the accompanying drawings. The invention is explained in more detail below using exemplary embodiments, which are specified in the schematic figures of the drawings, in which:
- Figs. 1A-1C: show a schematic diagram of an active fixation cardiac electrode lead according to a first embodiment of the invention;
- Figs. 2A-2C: show a schematic diagram of the active fixation cardiac electrode lead according to a second embodiment of the invention;
- Figs. 3A-3C: show a schematic diagram of the active fixation cardiac electrode lead according to a third embodiment of the invention;
- Figs. 4A-4C: show a schematic diagram of the active fixation cardiac electrode lead according to a fourth embodiment of the invention;
- Figs. 5A-5C: show a schematic diagram of the active fixation cardiac electrode lead according to a fifth embodiment of the invention;
- Figs. 6A-6C: show a schematic diagram of the active fixation cardiac electrode lead according to a sixth embodiment of the invention; and
- Figs. 7A-7C: show a schematic diagram of the active fixation cardiac electrode lead according to a seventh embodiment of the invention.

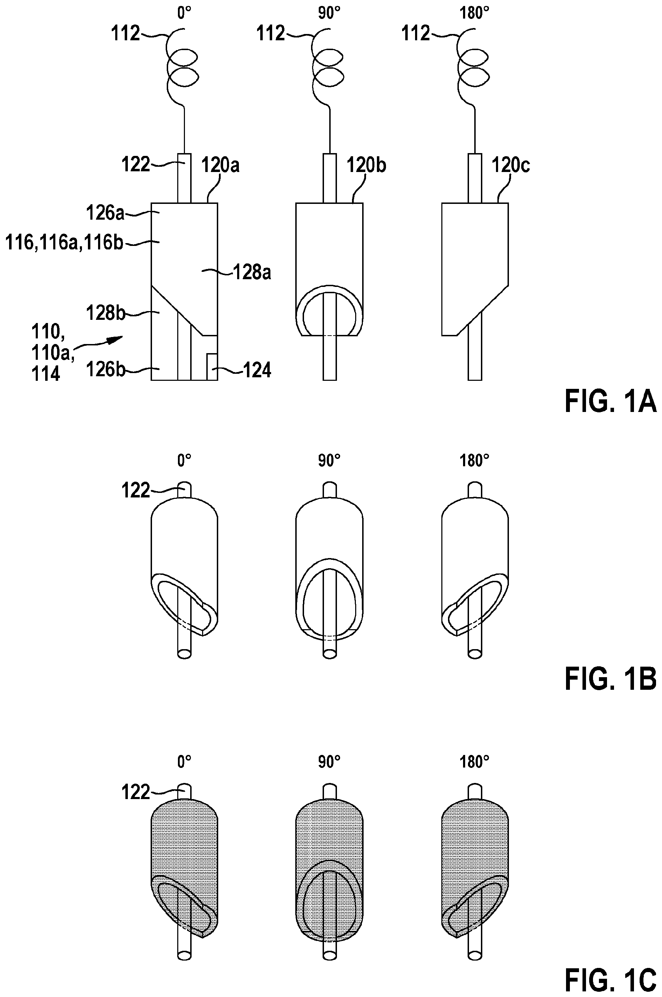

The active fixation cardiac electrode lead 110 shown in Figs. 1A, 1B, 1C comprises a fixation helix 112 movably or alternatively non-movably placed in a housing 114 of the electrode lead 110 at a distal end 110a of said electrode lead 110.

Fig.1A shows a side view of the electrode lead 110, Fig. 1B shows a perspective view of the electrode lead 110 and Fig. 1C shows a view of an x-ray image of the electrode lead 110.

The electrode lead 110 further comprises a first portion 116 of the electrode lead 110 having a first asymmetrical shape 116a and a first radiopacity 116b and a second portion 118 of the electrode lead 110 having a second asymmetrical shape 118a and a second radiopacity 118b, said second radiopacity 118b differing from said first radiopacity 116b, wherein the first portion 116 of the electrode lead 110 and/or the second portion 118 of the electrode lead 110 are adapted to indicate a rotational position 120a, 120b, 120c of the electrode lead 110 on an x-ray image during implantation of the electrode lead 110.

The first asymmetrical shape 116a of the first portion 116 and the second asymmetrical shape 118a of the second portion 118 are joined together to form the symmetrically, in particular a cylindrically shaped electrode lead 110. The first portion 116 of the electrode lead 110 is formed of at least one of platinum, a platinum-iridium alloy, silver, gold, iron, barium, strontium, cobalt, nickel, chromium, and tungsten.

The fixation helix 112 is fixed or alternatively rotatable relative to the housing 114 of the electrode lead 110. The first portion 116 of the electrode lead 110 and the second portion 118 of the electrode lead 110 surround an electrode body 122. Alternatively, only one of the first portion 116 of the electrode lead 110 and the second portion 118 of the electrode lead 110 may surround the electrode body 122.

The first portion 116 of the electrode lead 110 and the second portion 118 of the electrode lead 110 have an asymmetrical length. The electrode lead 110 further comprises a sensor 124 configured to detect a number of revolutions of the fixation helix 112.

Moreover, a first longitudinal section 126a, 126b of the first portion 116 the electrode lead 110 and the second portion 118 of the electrode lead 110 has a symmetrical, in particular cylindrical shape, and wherein a second longitudinal section 128a, 128b of the first portion 116 of the electrode lead 110 and the second portion 118 electrode lead 110 has an asymmetrical shape.

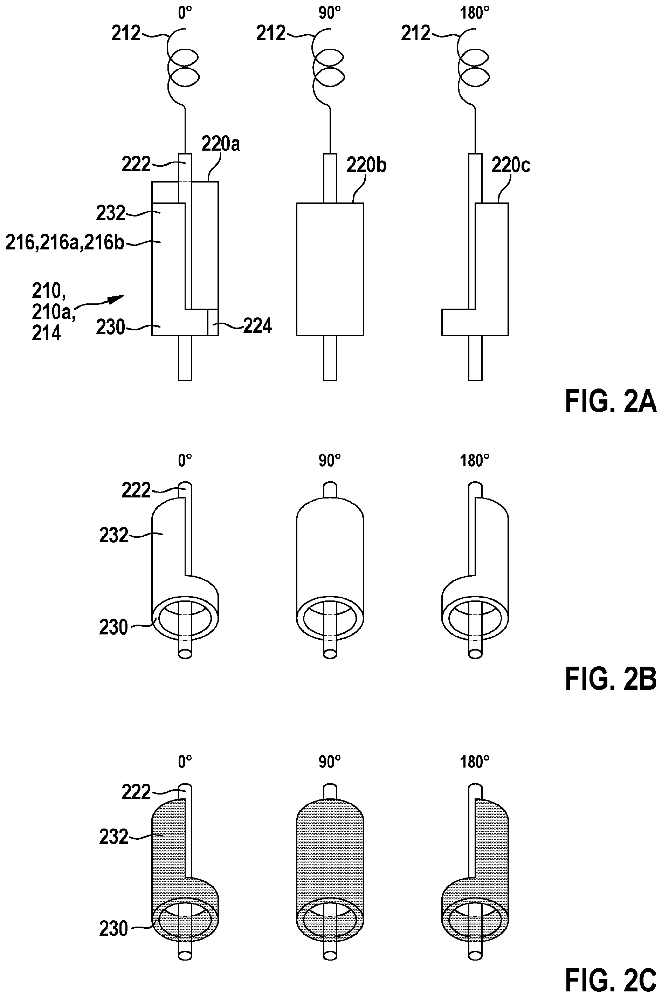

Figs. 2A, 2B, 2C show a schematic diagram of the active fixation cardiac electrode lead 210 according to a second embodiment of the invention. In particular, Fig.2A shows a side view of the electrode lead 210, Fig. 2B shows a perspective view of the electrode lead 210 and Fig. 2c shows a view of an x-ray image of the electrode lead 210.

A first portion 216 of the electrode lead 210 and/or a second portion 218 of the electrode lead 210 comprises a first ring shaped section 230 arranged at a distal end 210a of the electrode lead 210 and a second section 232 having a symmetric, in particular semi-cylindrical cutout.

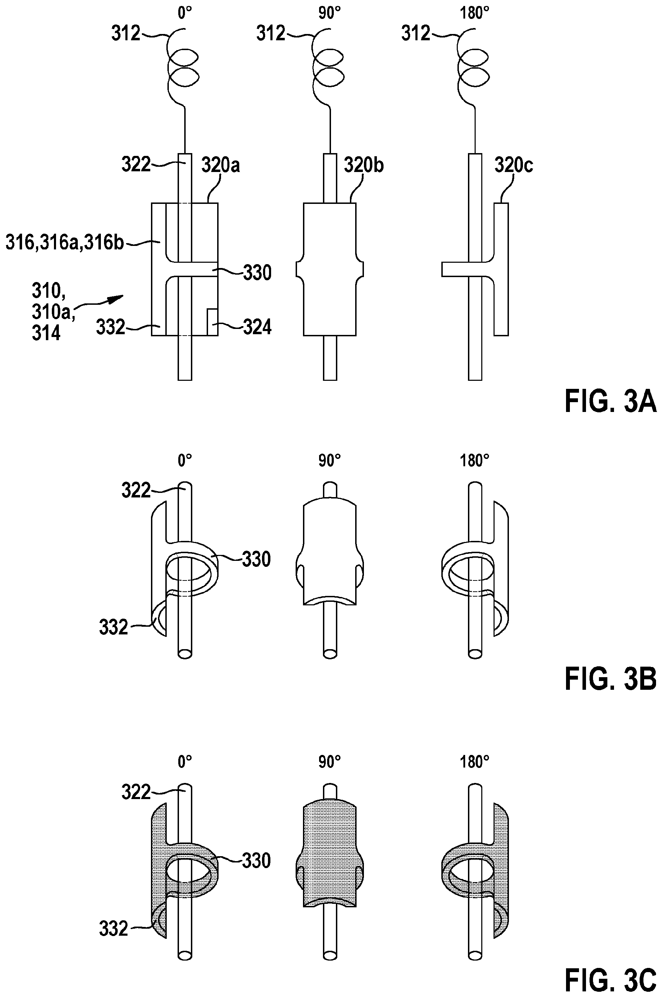

Figs. 3A, 3B, 3C show a schematic diagram of the active fixation cardiac electrode lead 310 according to a third embodiment of the invention. In particular, Fig.3A shows a side view of the electrode lead 310, Fig. 3B shows a perspective view of the electrode lead 310 and Fig. 3C shows a view of an x-ray image of the electrode lead 310.

A first portion 316 of the electrode lead 310 and/or a second portion 318 of the electrode lead 310 comprises a first ring shaped section 330 arranged at a longitudinal mid-section of the electrode lead 310 and a second section 332 having an asymmetric cutout arranged from the first ring shaped section 330 to each distal end 310a of the first portion 316 of the electrode lead 31 and/or a second portion 318 of the electrode lead 310 respectively.

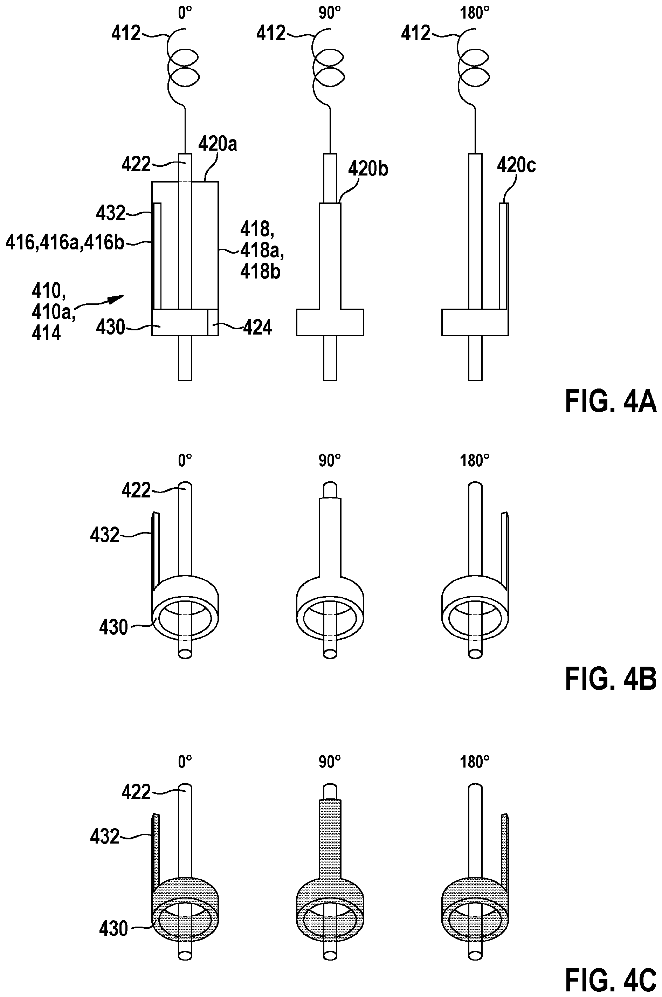

Figs. 4A, 4B, 4C show a schematic diagram of the active fixation cardiac electrode lead 410 according to a fourth embodiment of the invention. In particular, Fig.4A shows a side view of the electrode lead 410, Fig. 4B shows a perspective view of the electrode lead 410 and Fig. 4C shows a view of an x-ray image of the electrode lead 410.

A first portion 416 of the electrode lead 410 and/or a second portion 418 of the electrode lead 410 comprises a first ring shaped section 430 arranged at a distal end 410a of the electrode lead 410 and a second section 432 comprising a rod extending in a longitudinal direction of the electrode lead 410.

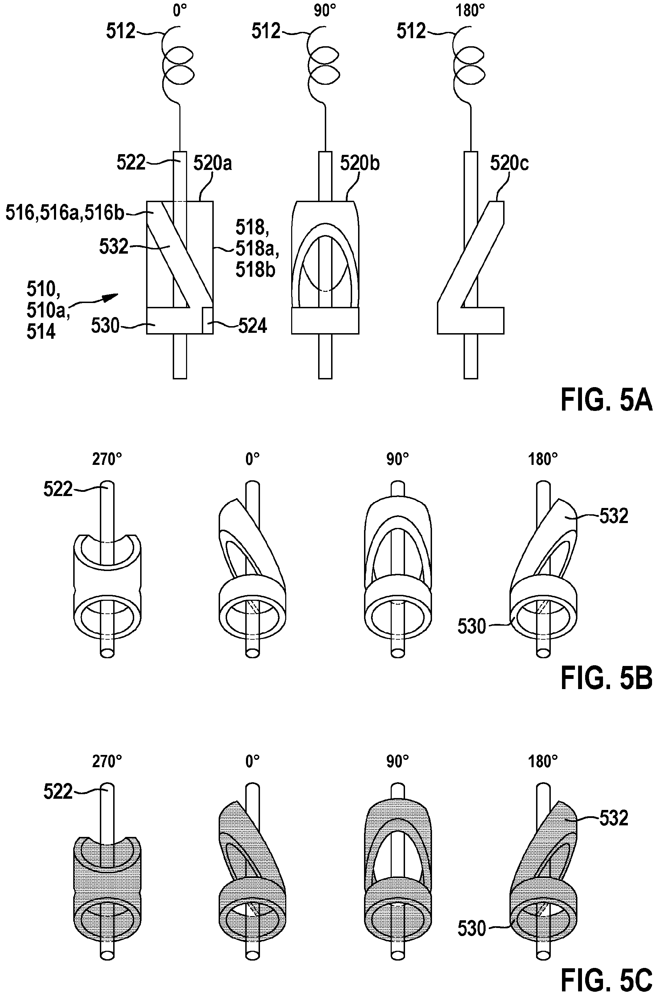

Figs. 5A, 5B, 5C show a schematic diagram of the active fixation cardiac electrode lead 510 according to a fifth embodiment of the invention. In particular, Fig.5A shows a side view of the electrode lead 510, Fig. 5B shows a perspective view of the electrode lead 510 and Fig. 5C shows a view of an x-ray image of the electrode lead 510.

A first portion 516 of the electrode lead 510 and/or a second portion 518 of the electrode lead 510 comprises a first ring shaped section 530 arranged at a distal end 510a of the electrode lead 510 and a second substantially elliptical ring-shaped section 532 being oriented oblique to a longitudinal axis of the electrode lead 510.

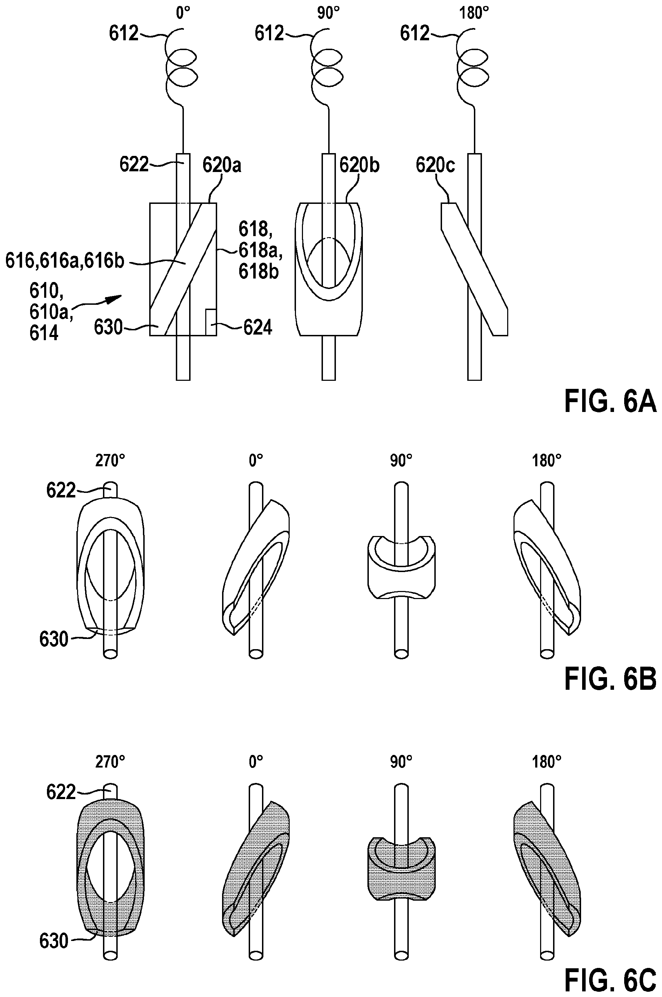

Figs. 6A, 6B, 6C show a schematic diagram of the active fixation cardiac electrode lead 610 according to a sixth embodiment of the invention. In particular, Fig.6A shows a side view of the electrode lead 610, Fig. 6B shows a perspective view of the electrode lead 610 and Fig. 6C shows a view of an x-ray image of the electrode lead 610.

A first portion 616 of the electrode lead 610 and/or a second portion 618 of the electrode lead 610 comprises a substantially elliptical ring-shaped section 630, a longer axis of which is oriented oblique to the longitudinal axis of the electrode lead 610.

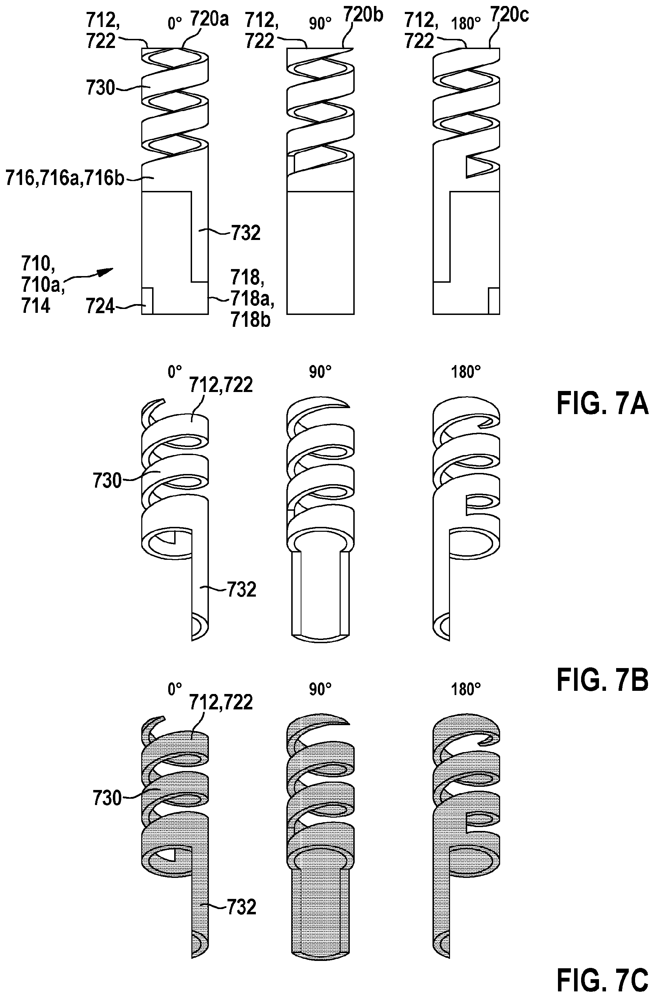

Figs. 7A, 7B, 7C show a schematic diagram of the active fixation cardiac electrode lead 710 according to a seventh embodiment of the invention. In particular, Fig.7A shows a side view of the electrode lead 710, Fig. 7B shows a perspective view of the electrode lead 710 and Fig. 7C shows a view of an x-ray image of the electrode lead 710.

The first asymmetrically shaped portion 716a of the electrode lead 710 or the second asymmetrically shaped portion 718a of the electrode lead 710 is integrally formed with the fixation helix 712 or the housing 714.

Furthermore, a first portion 716 of the electrode lead 710 and/or a second portion 718 of the electrode lead 710 comprises a first section 730 being formed by the fixation helix 712 and a second section 732 being integrally formed with the fixation helix 712, said second section having an asymmetric cutout arranged from the first section 730 to a distal end 710a of the first portion 716 of the electrode lead 710 and/or the second portion 718 of the electrode lead 710.

### Reference Signs

- 110; 210; 310; 410; 510; 610; 710: active fixation cardiac electrode lead
- 110a; 210a; 310a; 410a; 510a; 610a; 710a: distal end
- 112; 212; 312; 412; 512; 612; 712: fixation helix
- 114; 214; 314; 414; 514; 614; 714: housing
- 116; 216; 316; 416; 516; 616; 716: first portion
- 116a; 216a; 316a; 416a; 516a; 616a; 716a: first asymmetrical shape
- 116b; 216b; 316b; 416b; 516b; 616b; 716b: first radiopacity
- 118; 218; 318; 418; 518; 618; 718: second portion
- 118a; 218a; 318a; 418a; 518a; 618a; 718a: second asymmetrical shape
- 118b; 218b; 318b; 418b; 518b; 618b; 718b: second radiopacity
- 120a, 120b, 120c; 220a, 220b, 220c; 320a,: rotational position
- 320b, 320c; 420a, 420b, 420c; 520a, 520b,: rotational position
- 520c; 620a, 620b, 620c; 720a, 720b, 720c: rotational position
- 122; 222; 322; 422; 522; 622; 722: electrode body
- 124; 224; 324; 424; 524; 624; 724: sensor
- 126a, 126b: first longitudinal section
- 128a, 128b: second longitudinal section
- 230: first ring shaped section
- 232: second section
- 330: first ring shaped section
- 332: second section
- 430: first ring shaped section
- 432: second section
- 530: first ring shaped section
- 532: elliptical ring-shaped section
- 630: elliptical ring-shaped section
- 730: first section
- 732: second section

## Claims

1. Active fixation cardiac electrode lead (110; 210; 310; 410; 510; 610; 710) comprising:
a fixation helix (112; 212; 312; 412; 512; 612; 712) movably or non-movably placed in a housing (114; 214; 314; 414; 514; 614; 714) of the electrode lead (110; 210; 310; 410; 510; 610; 710) at a distal end (110a; 210a; 310a; 410a; 510a; 610a; 710a) of said electrode lead (110; 210; 310; 410; 510; 610; 710);
a first portion (116; 216; 316; 416; 516; 616; 716) of the electrode lead (110; 210; 310; 410; 510; 610; 710) having a first asymmetrical shape (116a; 216a; 316a; 416a; 516a; 616a; 716a) and a first radiopacity (116b; 216b; 316b; 416b; 516b; 616b; 716b); and
a second portion (118; 218; 318; 418; 518; 618; 718) of the electrode lead (110; 210; 310; 410; 510; 610; 710) having a second asymmetrical shape (118a; 218a; 318a; 418a; 518a; 618a; 718a) and a second radiopacity (118b; 218b; 318b; 418b; 518b; 618b; 718b), said second radiopacity (118b; 218b; 318b; 418b; 518b; 618b; 718b) differing from said first radiopacity (116b; 216b; 316b; 416b; 516b; 616b; 716b), wherein the first portion (116; 216; 316; 416; 516; 616; 716) of the electrode lead (110; 210; 310; 410; 510; 610; 710) and/or the second portion (118; 218; 318; 418; 518; 618; 718) of the electrode lead (110; 210; 310; 410; 510; 610; 710) are adapted to indicate a rotational position (120a, 120b, 120c; 220a, 220b, 220c; 320a, 320b, 320c; 420a, 420b, 420c; 520a, 520b, 520c; 620a, 620b, 620c; 720a, 720b, 720c) of the electrode lead (110; 210; 310; 410; 510; 610; 710) on an x-ray image during implantation of the electrode lead (110; 210; 310; 410; 510; 610; 710);
wherein the first portion (416) of the electrode lead (410) and/or the second portion (418) of the electrode lead (410) comprises a first ring shaped section (430) arranged at the distal end (410a) of the electrode lead (410) and a second section (432) comprising a rod extending in a longitudinal direction of the electrode lead (410).

2. Active fixation cardiac electrode lead of claim 1, wherein the first asymmetrically shaped portion (116a; 216a; 316a; 416a; 516a; 616a; 716a) of the electrode lead (110; 210; 310; 410; 510; 610; 710) or the second asymmetrically shaped portion (118a; 218a; 318a; 418a; 518a; 618a; 718a) of the electrode lead (110; 210; 310; 410; 510; 610; 710) is integrally formed with the fixation helix (112; 212; 312; 412; 512; 612; 712) or the housing (114; 214; 314; 414; 514; 614; 714).

3. Active fixation cardiac electrode lead of claim 1 or 2, wherein the first asymmetrical shape (116a; 216a; 316a; 416a; 516a; 616a; 716a) of the first portion (116; 216; 316; 416; 516; 616; 716) and the second asymmetrical shape (118a; 218a; 318a; 418a; 518a; 618a; 718a) of the second portion (118; 218; 318; 418; 518; 618; 718) are joined together to form the symmetrically, in particular a cylindrically shaped electrode lead (110; 210; 310; 410; 510; 610; 710).

4. Active fixation cardiac electrode lead of any one of the preceding claims, wherein the first portion (116; 216; 316; 416; 516; 616; 716) of the electrode lead (110; 210; 310; 410; 510; 610; 710) is formed of at least one of platinum, a platinum-iridium alloy, silver, gold, iron, barium, strontium, cobalt, nickel, chromium, and tungsten.

5. Active fixation cardiac electrode lead of any one of the preceding claims, wherein the fixation helix (112; 212; 312; 412; 512; 612; 712) is rotatable or fixed relative to the housing (114; 214; 314; 414; 514; 614; 714) of the electrode lead (110; 210; 310; 410; 510; 610; 710).

6. Active fixation cardiac electrode lead of any one of the preceding claims, wherein the first portion (116; 216; 316; 416; 516; 616; 716) of the electrode lead (110; 210; 310; 410; 510; 610; 710) and/or the second portion (118; 218; 318; 418; 518; 618; 718) of the electrode lead (110; 210; 310; 410; 510; 610; 710) surround an electrode body (122; 222; 322; 422; 522; 622; 722).

7. Active fixation cardiac electrode lead of any one of the preceding claims, wherein the first portion (116; 216; 316; 416; 516; 616; 716) of the electrode lead (110; 210; 310; 410; 510; 610; 710) and/or the second portion (118; 218; 318; 418; 518; 618; 718) of the electrode lead (110; 210; 310; 410; 510; 610; 710) has an asymmetrical length.

8. Active fixation cardiac electrode lead of any one of the preceding claims, wherein the electrode lead (110; 210; 310; 410; 510; 610; 710) comprises a sensor (124; 224; 324; 424; 524; 624; 724) configured to detect a number of revolutions of the fixation helix (112; 212; 312; 412; 512; 612; 712).

9. Active fixation cardiac electrode lead of any one of the preceding claims, wherein a first longitudinal section (126a, 126b) of the first portion (116) of the electrode lead (110) and the second portion (118) of the electrode lead (110) has a symmetrical, in particular cylindrical shape, and wherein a second longitudinal section (128a, 128b) of the first portion (116) of the electrode lead (110) and the second portion (118) electrode lead (110) has an asymmetrical shape.

10. Active fixation cardiac electrode lead of any one of claims 1 to 8, wherein the first portion (216) of the electrode lead (210) and/or the second portion (218) of the electrode lead (210) comprises a first ring shaped section (230) arranged at the distal end (210a) of the electrode lead (210) and a second section (232) having a symmetric, in particular semi-cylindrical cutout.

11. Active fixation cardiac electrode lead of any one of claims 1 to 8, wherein the first portion (316) of the electrode lead (310) and/or the second portion (318) of the electrode lead (310) comprises a first ring shaped section (330) arranged at a longitudinal mid-section of the electrode lead (310) and a second section (332) having an asymmetric cutout arranged from the first ring shaped section (330) to each distal end (310a) of the first portion (316) of the electrode lead (31) and/or the second portion (318) of the electrode lead (310) respectively.

12. Active fixation cardiac electrode lead of any one of claims 1 to 8, wherein the first portion (516) of the electrode lead (510) and/or the second portion (518) of the electrode lead (510) comprises a first ring shaped section (530) arranged at the distal end (510a) of the electrode lead (510) and a second substantially elliptical ring-shaped section (532) being oriented oblique to a longitudinal axis of the electrode lead (510).

13. Active fixation cardiac electrode lead of any one of claims 1 to 8, wherein the first portion (616) of the electrode lead (610) and/or the second portion (618) of the electrode lead (610) comprises a substantially elliptical ring-shaped section (630), a longer axis of which is oriented oblique to the longitudinal axis of the electrode lead (610).

14. Active fixation cardiac electrode lead of any one of claims 1 to 8, wherein the first portion (716) of the electrode lead (710) and/or the second portion (718) of the electrode lead (710) comprises a first section (730) being formed by the fixation helix (712) and a second section (732) being integrally formed with the fixation helix (712), said second section (732) having an asymmetric cutout arranged from the first section (730) to the distal end (710a) of the first portion (716) of the electrode lead (710) and/or the second portion (718) of the electrode lead (710).

## Patentansprüche

1. Aktive Fixierung der Herzelektrodenleitung (110; 210; 310; 410; 510; 610; 710), umfassend:
eine Fixierungswendel (112; 212; 312; 412; 512; 612; 712), die beweglich oder unbeweglich in einem Gehäuse (114; 214; 314; 414; 514; 614; 714) der Elektrodenleitung (110; 210; 310; 410; 510; 610; 710) an einem distalen Ende (110a; 210a; 310a; 410a; 510a; 610a; 710a) der Elektrodenleitung (110; 210; 310; 410; 510; 610; 710) angeordnet ist;
ein erster Teil (116; 216; 316; 416; 516; 616; 716) der Elektrodenleitung (110; 210; 310; 410; 510; 610; 710) mit einer ersten asymmetrischen Form (116a; 216a; 316a; 416a; 516a; 616a; 716a) und einer ersten Röntgendichte (116b; 216b; 316b; 416b; 516b; 616b; 716b) aufweist; und
ein zweiter Teil (118; 218; 318; 418; 518; 618; 718) der Elektrodenleitung (110; 210; 310; 410; 510; 610; 710) mit einer zweiten asymmetrischen Form (118a; 218a; 318a; 418a; 518a; 618a; 718a) und einer zweiten Röntgendichte (118b; 218b; 318b; 418b; 518b; 618b; 718b) aufweist, wobei sich die zweite Röntgendichte (118b; 218b; 318b; 418b; 518b; 618b; 718b) von der ersten Röntgendichte (116b; 216b; 316b; 416b; 516b; 616b; 716b) unterscheidet, wobei sich der erste Teil (116; 216; 316; 416; 516; 616; 716) der Elektrodenleitung (110; 210; 310; 410; 510; 610; 710) und/oder der zweite Teil (118; 218; 318; 418; 518; 618; 718) der Elektrodenleitung (110; 210; 310; 410; 510; 610; 710) sind so ausgelegt, dass sie eine Drehposition (120a, 120b, 120c; 220a, 220b, 220c; 320a, 320b, 320c; 420a, 420b, 420c; 520a, 520b, 520c; 620a, 620b, 620c; 720a, 720b, 720c) der Elektrodenleitung (110; 210; 310; 410; 510; 610; 710) auf einem Röntgenbild während der Implantation der Elektrodenleitung (110; 210; 310; 410; 510; 610; 710)
wobei der erste Teil (416) der Elektrodenleitung (410) und/oder der zweite Teil (418) der Elektrodenleitung (410) einen ersten ringförmigen Abschnitt (430), der am distalen Ende (410a) der Elektrodenleitung (410) angeordnet ist, und einen zweiten Abschnitt (432) umfasst, der einen sich in Längsrichtung der Elektrodenleitung erstreckenden Stab umfasst (410) erstreckt.

2. Aktive Fixierung der Herzelektrodenleitung nach Anspruch 1, wobei der erste asymmetrisch geformte Teil (116a; 216a; 316a; 416a; 516a; 616a; 716a) der Elektrodenleitung (110; 210; 310; 410; 510; 610; 710) oder der zweite asymmetrisch geformte Teil (118a; 218a; 318a; 418a; 518a; 618a; 718a) der Elektrodenleitung (110; 210; 310; 410; 510; 610; 710) ist integral mit der Fixierungswendel (112; 212; 312; 412; 512; 612; 712) oder dem Gehäuse (114; 214; 314; 414; 514; 614; 714) ausgebildet.

3. Aktive Fixierung der Herzelektrodenleitung nach Anspruch 1 oder 2, wobei die erste asymmetrische Form (116a; 216a; 316a; 416a; 516a; 616a; 716a) des ersten Teils (116; 216; 316; 416; 516; 616; 716) und die zweite asymmetrische Form (118a; 218a; 318a; 418a; 518a; 618a; 718a) des zweiten Teils (118; 218; 318; 418; 518; 618; 718) sind miteinander verbunden, um die symmetrische, insbesondere zylindrisch geformte Elektrodenleitung (110; 210; 310; 410; 510; 610; 710) zu bilden.

4. Aktive Fixierung der Herzelektrodenleitung nach einem der vorstehenden Ansprüche, wobei der erste Teil (116; 216; 316; 416; 516; 616; 716) der Elektrodenleitung (110; 210; 310; 410; 510; 610; 710) aus mindestens einem der folgenden Materialien gebildet ist: Platin, einer Platin-Iridium-Legierung, Silber, Gold, Eisen, Barium, Strontium, Kobalt, Nickel, Chrom und Wolfram.

5. Aktive Fixierung der Herzelektrodenleitung gemäß einem der vorstehenden Ansprüche, wobei die Fixierungswendel (112; 212; 312; 412; 512; 612; 712) relativ zum Gehäuse (114; 214; 314; 414; 514; 614; 714) der Elektrodenleitung (110; 210; 310; 410; 510; 610; 710) drehbar oder feststehend ist.

6. Aktive Fixierung der Herzelektrodenleitung gemäß einem der vorstehenden Ansprüche, wobei der erste Teil (116; 216; 316; 416; 516; 616; 716) der Elektrodenleitung (110; 210; 310; 410; 510; 610; 710) und/oder der zweite Teil (118; 218; 318; 418; 518; 618; 718) der Elektrodenleitung (110; 210; 310; 410; 510; 610; 710) einen Elektrodenkörper (122; 222; 322; 422; 522; 622; 722) umgeben.

7. Aktive Fixierung der Herzelektrodenleitung nach einem der vorstehenden Ansprüche, wobei der erste Teil (116; 216; 316; 416; 516; 616; 716) der Elektrodenleitung (110; 210; 310; 410; 510; 610; 710) und/oder der zweite Teil (118; 218; 318; 418; 518; 618; 718) der Elektrodenleitung (110; 210; 310; 410; 510; 610; 710) eine asymmetrische Länge aufweist.

8. Aktive Fixierung der Herzelektrodenleitung nach einem der vorstehenden Ansprüche, wobei die Elektrodenleitung (110; 210; 310; 410; 510; 610; 710) einen Sensor (124; 224; 324; 424; 524; 624; 724) umfasst, der so konfiguriert ist, dass er eine Anzahl von Windungen der Fixierungswendel (112; 212; 312; 412; 512; 612; 712) erfasst.

9. Aktive Fixierung der Herzelektrodenleitung nach einem der vorstehenden Ansprüche, wobei ein erster Längsabschnitt (126a, 126b) des ersten Teils (116) der Elektrodenleitung (110) und der zweite Teil (118) der Elektrodenleitung (110) eine symmetrische, insbesondere zylindrische Form aufweisen, und wobei ein zweiter Längsabschnitt (128a, 128b) des ersten Teils (116) der Elektrodenleitung (110) und des zweiten Teils (118) der Elektrodenleitung (110) eine asymmetrische Form aufweist.

10. Aktive Fixierung der Herzelektrodenleitung nach einem der Ansprüche 1 bis 8, wobei der erste Teil (216) der Elektrodenleitung (210) und/oder der zweite Teil (218) der Elektrodenleitung (210) einen ersten ringförmigen Abschnitt (230), der am distalen Ende (210a) der Elektrodenleitung (210) angeordnet ist, und einen zweiten Abschnitt (232) mit einer symmetrischen, insbesondere halbzylindrischen Aussparung aufweist.

11. Aktive Fixierung der Herzelektrodenleitung nach einem der Ansprüche 1 bis 8, wobei der erste Teil (316) der Elektrodenleitung (310) und/oder der zweite Teil (318) der Elektrodenleitung (310) einen ersten ringförmigen Abschnitt (330), der in einem Längsmittelabschnitt der Elektrodenleitung (310) angeordnet ist, und einen zweiten Abschnitt (332) mit einem asymmetrischen Ausschnitt aufweist, der vom ersten ringförmigen Abschnitt (330) zu jedem distalen Ende (310a) des ersten Teils (316) der Elektrodenleitung (31) und/oder des zweiten Teils (318) der Elektrodenleitung (310) angeordnet ist.

12. Aktive Fixierung der Herzelektrodenleitung nach einem der Ansprüche 1 bis 8, wobei der erste Teil (516) der Elektrodenleitung (510) und/oder der zweite Teil (518) der Elektrodenleitung (510) einen ersten ringförmigen Abschnitt (530), der am distalen Ende (510a) der Elektrodenleitung (510) angeordnet ist, und einen zweiten im Wesentlichen elliptischen ringförmigen Abschnitt (532) umfasst, der schräg zu einer Längsachse der Elektrodenleitung (510) ausgerichtet ist.

13. Aktive Fixierung der Herzelektrodenleitung nach einem der Ansprüche 1 bis 8, wobei der erste Teil (616) der Elektrodenleitung (610) und/oder der zweite Teil (618) der Elektrodenleitung (610) einen im Wesentlichen elliptischen ringförmigen Abschnitt (630) umfasst, dessen längere Achse schräg zur Längsachse der Elektrodenleitung (610) ausgerichtet ist.

14. Aktive Fixierung der Herzelektrodenleitung nach einem der Ansprüche 1 bis 8, wobei der erste Teil (716) der Elektrodenleitung (710) und/oder der zweite Teil (718) der Elektrodenleitung (710) einen ersten Abschnitt (730), der durch die Fixierungswendel (712) gebildet wird, und einen zweiten Abschnitt (732) umfasst, der integral mit der Fixierungswendel (712) ausgebildet ist, wobei der zweite Abschnitt (732) einen asymmetrischen Ausschnitt aufweist, der vom ersten Abschnitt (730) zum distalen Ende (710a) des ersten Teils (716) der Elektrodenleitung (710) und/oder des zweiten Teils (718) der Elektrodenleitung (710).

## Revendications

1. Câble d'électrode cardiaque à fixation active (110 ; 210 ; 310 ; 410 ; 510 ; 610 ; 710) comprenant :
une spirale de fixation (112 ; 212 ; 312 ; 412 ; 512 ; 612 ; 712) placée de manière mobile ou non mobile dans un boîtier (114 ; 214 ; 314 ; 414 ; 514 ; 614 ; 714) du câble d'électrode (110 ; 210 ; 310 ; 410 ; 510 ; 610 ; 710) au niveau d'une extrémité distale (110a ; 210a ; 310a ; 410a ; 510a ; 610a; 710a) dudit câble d'électrode (110 ; 210 ; 310 ; 410 ; 510 ; 610 ; 710) ;
une première partie (116 ; 216 ; 316 ; 416 ; 516 ; 616 ; 716) du câble d'électrode (110 ; 210 ; 310 ; 410 ; 510 ; 610 ; 710) ayant une première forme asymétrique (116a ; 216a ; 316a ; 416a ; 516a ; 616a ; 716a) et une première radio-opacité (116b ; 216b ; 316b ; 416b ; 516b ; 616b ; 716b) ; et
une seconde partie (118 ; 218 ; 318 ; 418 ; 518 ; 618 ; 718) du câble d'électrode (110 ; 210 ; 310 ; 410 ; 510 ; 610 ; 710) ayant une seconde forme asymétrique (118a ; 218a ; 318a ; 418a ; 518a ; 618a ; 718a) et une seconde radio-opacité (118b ; 218b ; 318b ; 418b ; 518b ; 618b ; 718b), ladite seconde radio-opacité (118b ; 218b ; 318b ; 418b ; 518b ; 618b ; 718b) différant de ladite première radio-opacité (116b ; 216b ; 316b ; 416b ; 516b ; 616b ; 716b), dans lequel la première partie (116 ; 216 ; 316 ; 416 ; 516 ; 616 ; 716) du câble d'électrode (110 ; 210 ; 310 ; 410 ; 510 ; 610 ; 710) et/ou la seconde partie (118 ; 218 ; 318 ; 418 ; 518 ; 618 ; 718) du câble d'électrode (110 ; 210 ; 310 ; 410 ; 510 ; 610 ; 710) sont adaptées pour indiquer une position de rotation (120a, 120b, 120c ; 220a, 220b, 220c ; 320a, 320b, 320c ; 420a, 420b, 420c ; 520a, 520b, 520c ; 620a, 620b, 620c ; 720a, 720b, 720c) du câble d'électrode (110 ; 210 ; 310 ; 410 ; 510 ; 610 ; 710) sur une image aux rayons X pendant l'implantation du câble d'électrode (110 ; 210 ; 310 ; 410 ; 510 ; 610 ; 710) ;
dans lequel la première partie (416) du câble d'électrode (410) et/ou la seconde partie (418) du câble d'électrode (410) comprend une première section de forme annulaire (430) agencée au niveau de l'extrémité distale (410a) du câble d'électrode (410) et une seconde section (432) comprenant une tige s'étendant dans une direction longitudinale du câble d'électrode (410).

2. Câble d'électrode cardiaque à fixation active selon la revendication 1, dans lequel la première partie de forme asymétrique (116a ; 216a ; 316a ; 416a ; 516a ; 616a ; 716a) du câble d'électrode (110 ; 210 ; 310 ; 410 ; 510 ; 610 ; 710) ou la seconde partie de forme asymétrique (118a ; 218a; 318a; 418a; 518a ; 618a ; 718a) du câble d'électrode (110 ; 210 ; 310 ; 410 ; 510 ; 610 ; 710) est formée de manière intégrale avec la spirale de fixation (112 ; 212 ; 312 ; 412 ; 512 ; 612 ; 712) ou le boîtier (114 ; 214 ; 314 ; 414 ; 514 ; 614 ; 714).

3. Câble d'électrode cardiaque à fixation active selon la revendication 1 ou 2, dans lequel la première forme asymétrique (116a ; 216a ; 316a ; 416a ; 516a ; 616a ; 716a) de la première partie (116 ; 216 ; 316 ; 416 ; 516 ; 616 ; 716) et la seconde forme asymétrique (118a ; 218a ; 318a ; 418a ; 518a ; 618a ; 718a) de la seconde partie (118 ; 218 ; 318 ; 418 ; 518 ; 618 ; 718) sont joints ensemble pour former le câble d'électrode de forme symétrique, en particulier un câble d'électrode de forme cylindrique (110 ; 210 ; 310 ; 410 ; 510 ; 610 ; 710).

4. Câble d'électrode cardiaque à fixation active selon l'une quelconque des revendications précédentes, dans lequel la première partie (116 ; 216 ; 316 ; 416 ; 516 ; 616 ; 716) du câble d'électrode (110 ; 210 ; 310 ; 410 ; 510 ; 610 ; 710) est formée d'au moins un parmi le platine, un alliage platine-iridium, l'argent, l'or, le fer, le baryum, le strontium, le cobalt, le nickel, le chrome et le tungstène.

5. Câble d'électrode cardiaque à fixation active selon l'une quelconque des revendications précédentes, dans lequel la spirale de fixation (112 ; 212 ; 312 ; 412 ; 512 ; 612 ; 712) peut tourner ou est fixe par rapport au boîtier (114 ; 214 ; 314 ; 414 ; 514 ; 614 ; 714) du câble d'électrode (110 ; 210 ; 310 ; 410 ; 510 ; 610 ; 710).

6. Câble d'électrode cardiaque à fixation active selon l'une quelconque des revendications précédentes, dans lequel la première partie (116 ; 216 ; 316 ; 416 ; 516 ; 616 ; 716) du câble d'électrode (110 ; 210 ; 310 ; 410 ; 510 ; 610 ; 710) et/ou la seconde partie (118 ; 218 ; 318 ; 418 ; 518 ; 618 ; 718) du câble d'électrode (110 ; 210 ; 310 ; 410 ; 510 ; 610 ; 710) entoure un corps d'électrode (122 ; 222 ; 322 ; 422 ; 522 ; 622 ; 722).

7. Câble d'électrode cardiaque à fixation active selon l'une quelconque des revendications précédentes, dans lequel la première partie (116 ; 216 ; 316 ; 416 ; 516 ; 616 ; 716) du câble d'électrode (110 ; 210 ; 310 ; 410 ; 510 ; 610 ; 710) et/ou la seconde partie (118 ; 218 ; 318 ; 418 ; 518 ; 618 ; 718) du câble d'électrode (110 ; 210 ; 310 ; 410 ; 510 ; 610 ; 710) a une longueur asymétrique.

8. Câble d'électrode cardiaque à fixation active selon l'une quelconque des revendications précédentes, dans lequel le câble d'électrode (110 ; 210 ; 310 ; 410 ; 510 ; 610 ; 710) comprend un capteur (124 ; 224 ; 324 ; 424 ; 524 ; 624 ; 724) configuré pour détecter un nombre de révolutions de la spirale de fixation (112 ; 212 ; 312 ; 412 ; 512 ; 612 ; 712).

9. Câble d'électrode cardiaque à fixation active selon l'une quelconque des revendications précédentes, dans lequel une première section longitudinale (126a, 126b) de la première partie (116) du câble d'électrode (110) et de la seconde partie (118) du câble d'électrode (110) a une forme symétrique, en particulier cylindrique, et dans lequel une seconde section longitudinale (128a, 128b) de la première partie (116) du câble d'électrode (110) et de la seconde partie (118) du câble d'électrode (110) a une forme asymétrique.

10. Câble d'électrode cardiaque à fixation active selon l'une quelconque des revendications 1 à 8, dans lequel la première partie (216) du câble d'électrode (210) et/ou la seconde partie (218) du câble d'électrode (210) comprend une première section de forme annulaire (230) agencée au niveau de l'extrémité distale (210a) du câble d'électrode (210) et une seconde section (232) ayant un ajour symétrique, en particulier semi-cylindrique.

11. Câble d'électrode cardiaque à fixation active selon l'une quelconque des revendications 1 à 8, dans lequel la première partie (316) du câble d'électrode (310) et/ou la seconde partie (318) du câble d'électrode (310) comprend une première section de forme annulaire (330) agencée au niveau d'une section médiane longitudinale du câble d'électrode (310) et une seconde section (332) ayant un ajour asymétrique agencé à partir de la première section de forme annulaire (330) jusqu'à respectivement chaque extrémité distale (310a) de la première partie (316) du câble d'électrode (31) et/ou de la seconde partie (318) du câble d'électrode (310).

12. Câble d'électrode cardiaque à fixation active selon l'une quelconque des revendications 1 à 8, dans lequel la première partie (516) du câble d'électrode (510) et/ou la seconde partie (518) du câble d'électrode (510) comprend une première section de forme annulaire (530) agencée au niveau de l'extrémité distale (510a) du câble d'électrode (510) et une seconde section de forme annulaire essentiellement elliptique (532) orientée de manière oblique vis-à-vis d'un axe longitudinal du câble d'électrode (510).

13. Câble d'électrode cardiaque à fixation active selon l'une quelconque des revendications 1 à 8, dans lequel la première partie (616) du câble d'électrode (610) et/ou la seconde partie (618) du câble d'électrode (610) comprend une section de forme annulaire essentiellement elliptique (630), dont un axe plus long est orienté de manière oblique vis-à-vis de l'axe longitudinal du câble d'électrode (610).

14. Câble d'électrode cardiaque à fixation active selon l'une quelconque des revendications 1 à 8, dans lequel la première partie (716) du câble d'électrode (710) et/ou la seconde partie (718) du câble d'électrode (710) comprend une première section (730) formée par la spirale de fixation (712) et une seconde section (732) formée de manière intégrale avec la spirale de fixation (712), ladite seconde section (732) ayant un ajour asymétrique agencé depuis la première section (730) jusqu'à l'extrémité distale (710a) de la première partie (716) du câble d'électrode (710) et/ou de la seconde partie (718) du câble d'électrode (710).
